# EUROPÄISCHE PATENTANMELDUNG

(11) **EP 2 198 801 A1**
(43) Veröffentlichungstag der Anmeldung: **23.06.2010**
(21) Anmeldenummer: 08172335.5
(22) Anmeldetag: 19.12.2008
(51) Int. Cl.: A61B 19/00

(54) **Marker-Anbringvorrichtung zum Anbringen einer Markervorrichtung**

(71) Anmelder: BrainLAB AG, 85622 Feldkirchen (DE)
(72) Erfinder: Gschwandtner, Michael, 80469, München (DE); Kilian, Antonia, 85570, Markt Schwaben (DE); Frimor, Troels, 81667, München (DE)
(74) Vertreter: Schwabe - Sandmair - Marx

(57) **Zusammenfassung**

Die vorliegende Anmeldung betrifft eine Marker-Anbringvorrichtung (6) zum Anbringen einer Markervorrichtung (21) an einem Gegenstand, insbesondere an einem medizinischen Instrument (3) und/oder einer anatomischen Körperstruktur (14) und/oder einem Implantat (14), mit einer Befestigungsmöglichkeit (2) für eine Markervorrichtung (21) und einem Anbringteil (1) zum Anbringen der Marker-Anbringvorrichtung (6), wobei das Anbringteil (1) verformbar ist, um mit dem Gegenstand eine mechanische Verbindung einzugehen.

## Beschreibung

Die vorliegende Erfindung betrifft eine Marker-Anbringvorrichtung zum Anbringen einer Markervorrichtung an einem Gegenstand, insbesondere an einem medizinischen Instrument und/oder einer anatomischen Körperstruktur und/oder einem Implantat, mit einer Befestigungsmöglichkeit für eine Markervorrichtung und mit einem Anbringteil zum Anbringen der Marker-Anbringvorrichtung an den Gegenstand.

Moderne Operationsverfahren im Bereich der Image Guided Surgery (IGS) werden häufig mit Hilfe eines medizintechnischen Navigations- bzw. Trackingsystems durchgeführt. Die Navigation basiert zumeist auf dem Vergleich wenigstens eines mehrdimensionalen Modells von wenigstens Teilen des Patientenkörpers mit während des medizinischen Eingriffs erfassten Daten von wenigstens Teilen des Patientenkörpers, wobei der Referenzdatensatz, der Daten über wenigstens die Geometrie wenigstens eines Teils des Patientenkörpers beinhalten kann und auf dem das Modell basiert, mithilfe eines medizintechnischen bildgebenden Verfahrens vor der Operation aufgezeichnet wird. Ferner kann ein Referenzdatensatz mit geometrischen Informationen über ein Implantat und/oder ein medizinisches Instrument bereitgestellt werden. Eine an einer anatomischen Körperstruktur des Patienten und/oder einem medizinischen Instrument und/oder einem Implantat (im Folgenden werden alle drei als Gegenstand bezeichnet, die Begriffe der anatomischen Körperstruktur, des medizinischen Instruments bzw. Implantats sollen im Rahmen dieses Textes austauschbar bezüglich ihrer Kombination mit der erfindungsgemäßen Marker-Anbringvorrichtung sein) während der Operation angebrachte Markervorrichtung dient dann dazu, die aktuelle Lage des Gegenstandes mit dem Referenzdatensatz des Patientenkörpers und/oder dem Referenzdatensatz des Instruments und/oder Implantats zu vergleichen und somit die Navigation während der Operation zu ermöglichen.

Die US 6,190,395 B1 zeigt ein Anbringteil für ein chirurgisches Navigationssystem mit einem ringförmigen Anbringteil für eine Markervorrichtung. Gemäß Anspruch 3 dieser Schrift ist das ringförmige Befestigungsband aus einer Edelstahlfolie mit einer Dicke zwischen etwa 5 und 50 mm hergestellt. Diese Ausgestaltung erlaubt keine universelle Anpassbarkeit an eine Instrumentenoberfläche oder die Oberfläche einer anatomischen Struktur.

Die US 6,434,507 B1 zeigt einen Adapter für einen Instrumentenkopf, mit dem der Instrumentenkopf an einen Handgriff angebracht werden kann. An das vom Adapter entfernt liegende Ende des Instrumentes kann dann eine Markervorrichtung angebracht werden. Der Unterschied zum erfindungsgemäßen Gegenstand liegt hier zum Einen darin, dass zwischen die Marker-Anbringvorrichtung (d.h., den Adapter) und die Markervorrichtung ein für die Durchführung der medizinischen Aufgabe notwendiger Bestandteil des Instrumentes (der Instrumentenkopf) eingefügt wird. Außerdem ist hier das Anbringteil nicht offensichtlich universell anpassbar.

Aufgabe der Erfindung ist es, bei diesem Verfahren eine Markervorrichtung an wenigstens einem der genannten Gegenstände zu befestigen, um die Navigation zu ermöglichen, obwohl keine Markervorrichtung in wenigstens einen der Gegenstände integriert ist bzw. keine spezielle Halterung an wenigstens einem der Gegenstände vorgesehen ist.
Vorstehende Aufgabe wird durch die Gegenstände der unabhängigen Ansprüche gelöst. Die abhängigen Ansprüche sind auf vorteilhafte Weiterbildungen dieser Gegenstände gerichtet.

Die erfindungsgemäße Marker-Anbringvorrichtung ist zum Anbringen einer Markervorrichtung an einem Gegenstand, insbesondere an einem medizinischen Instrument und/oder einer anatomischen Körperstruktur und/oder einem Implantat, ausgebildet. Vorzugsweise ist die Marker-Anbringvorrichtung mit einer Befestigungsmöglichkeit für eine Markervorrichtung und einem Anbringteil zum Anbringen der Marker-Anbringvorrichtung an den Gegenstand versehen. Vorzugsweise ist das Anbringteil verformbar, insbesondere an wenigstens eine gegebene Form des Gegenstands anpassbar oder anformbar, so dass die Anbringvorrichtung bzw. das Anbringteil eine mechanische Verbindung mit dem Gegenstand eingeht. Insbesondere ist das Anbringteil so verformbar, dass es an einer Oberfläche des Gegenstands anliegt. Eine mechanische Verbindung bzw. eine Bindung, z.B. eine formschlüssige Verbindung des Anbringteils mit dem Gegenstand kann dadurch unterstützt werden, dass der Umfang des Anbringteils entlang seiner dem Gegenstand zugewandten Oberfläche im angebrachten Zustand ungefähr gleich dem Umfang wenigstens eines Teils des Gegenstandes ist, an den das Anbringteil angebracht ist, so dass das Anbringteil gerade wenigstens einen Teil des Gegenstands umgreift. Dadurch ist eine universelle Anpassbarkeit des Anbringteils an Gegenstände unterschiedlicher Geometrie möglich. Das Anbringteil stellt also eine insbesondere ortsfeste Verbindung zwischen einer Markervorrichtung und beliebig geformten Gegenständen her, selbst wenn diese ursprünglich nicht für die Kombination mit einer Markervorrichtung vorgesehen sind. Insofern ist das Anbringteil für eine mechanische Verbindung mit dem Gegenstand ausgebildet. Die erfindungsgemäße Anbringvorrichtung dient also insbesondere als ein Adapter zum Anbringen einer Markervorrichtung an eine Vielzahl unterschiedlich geformter Gegenstände. Das Anbringteil kann durch plastische Verformung an den Gegenstand anbringbar und eventuell von diesem lösbar sein. Die Befestigungsmöglichkeit kann beispielsweise ein Bestandteil der Anbringvorrichtung, z.B. eine Kante, ein Fortsatz oder der Rand eines Loches sein, wobei sich an dem Bestandteil die Markervorrichtung anbringen lässt. Die Befestigungsmöglichkeit kann auch eine an das Anbringteil angefügt mechanische Halteverbindung zur mechanischen Verbindung der Marker-Anbringvorrichtung mit der Markervorrichtung beinhalten, die für eine mechanische (z.B. kraftschlüssige und/oder reibschlüssige und/oder stoffschlüssige- und/oder formschlüssige, ortsfeste und/oder lösbare) Verbindung bzw. eine Bindung mit der Markervorrichtung und/oder dem Anbringteil ausgebildet ist und die z.B. Schnapp-, Rast-, Schraubverbindung, Einrastelemente, Gewinde usw. umfasst. Der Begriff der mechanischen Verbindung bzw. der Bindung beinhaltet im Rahmen dieser Erfindung eine kraftschlüssige, und/oder reibschlüssige und/oder formschlüssige und/oder stoffschlüssige Verbindung.

Vorzugsweise ist das Anbringteil aus Material hergestellt, das in der Rohform und/oder im angeformten Zustand mechanisch starr bzw. fest ist und unter der Einwirkung von Energie, die beispielsweise aus physikalischen und/oder chemischen Verfahren, insbesondere mechanischen Schwingungen bzw. Wellen und/oder thermischer Energie und/oder elektromagnetischer Strahlung und/oder bestimmten chemischen Reaktionen stammen kann, also unter externer Energieeinwirkung oder Energiefreisetzung, leichter, d.h. mit geringerem Kraftaufwand als in der Rohform und/oder im angeformten Zustand, plastisch verformbar wird (also verformbarer als vor der Energieeinwirkung). Die Verformung kann mit von einer menschlichen Hand ohne weitere Hilfsmittel erzielbaren Kräften, also z.B. mit bloßen Händen mit Kräften, deren Untergrenze bei etwa 0,1 N, 1 N, 10 N und/oder deren Obergrenze bei etwa 10 N*,* 100 N, 1000 N liegt, durchgeführt werden. Dadurch ist das Anbringteil auch unter OP-Bedingungen schnell und einfach ohne weitere Hilfsmittel und besondere technische Kenntnisse anbringbar.

Gemäß einer Ausführungsform der Erfindung ist das Anbringteil aus Material hergestellt, das in der Rohform leicht, d.h. mit von einer menschlichen Hand ohne weitere Hilfsmittel erzielbaren Kräften, also z.B. mit bloßen Händen mit Kräften, deren Untergrenze bei etwa 0,1 N, 1 N oder 10 N und/oder deren Obergrenze bei etwa 10 N, 100 N oder 1000 N liegt, plastisch verformbar ist. Im angeformten Zustand ist das Material des Anbringteils mechanisch starr bzw. fest. Unter der Einwirkung von Energie, die beispielsweise physikalische und/oder chemische Verfahren, insbesondere mechanische Schwingungen bzw. Wellen und/oder thermische Energie und/oder elektromagnetische Strahlung und/oder bestimmte chemischer Reaktionen beinhalten kann, also unter externer Energieeinwirkung oder Energiefreisetzung, wird es schwerer verformbar oder nicht verformbar, also nimmt es z.B. einen härteren Härtegrad an. Das heißt, es wird nur mit größerem Kraftaufwand als in der Rohform bzw. vor der Energieeinwirkung, der insbesondere oberhalb der oben genannten Obergrenzen liegt, plastisch verformbarer. Dadurch kann ein unter OP-Bedingungen realisierbares Verfahren genutzt werden, um einen festen Halt durch ein mechanisch stabiles Anbringteil zu erreichen.

Der Halt der Marker-Anbringvorrichtung an dem Gegenstand kann durch eine mechanische Verbindung bzw. eine Bindung insbesondere formschlüssig und/oder stoffschlüssig und/oder kraftschlüssig und/oder reibschlüssig, ortsfest und/oder von dem Gegenstand lösbar ausgebildet sein.

Das Anbringteil ist vorzugsweise so verformbar, dass es nach der Verformung den Gegenstand zumindest teilweise umgibt, um das gewünschte Anbringen zu erzielen. Umgibt das Anbringteil den Gegenstand vollständig, so ist die Oberfläche des Anbringteils geschlossen, während die Oberfläche den Gegenstand umgibt. Seine Oberfläche ist aber vorzugsweise an wenigstens einer Stelle unterbrochen, so dass ein Hohlraum im Inneren des Anbringteils entsteht, in dem sich der Gegenstand befindet, wobei der Hohlraum eine Verbindung zur umgebenden Atmosphäre durch die Unterbrechung der Oberfläche besitzt. Das Anbringteil hat insbesondere also mindestens eine und vorzugsweise zwei Außenkanten, die in sich geschlossen verlaufen und zwischen denen sich eine nach außen weisende Außenoberfläche (die im an den Gegenstand angebrachten Zustand vom Gegenstand weg weist) und eine nach innen weisende Innenoberfläche (die im an den Gegenstand angebrachten Zustand auf den Gegenstand weist) erstreckt. Diese bevorzugte Form soll fortan als "offene Form" bezeichnet werden, da sie mindestens eine Öffnung bereitstellt, die durch eine in sich geschlossene Außenkante definiert ist und durch die der Gegenstand (aus der Sicht des Anbringteils) nach außen vorstehen kann. Insbesondere im Fall von zwei Außenkanten können diese Außenkanten auch unterbrochen sein, so dass auch entsprechend die Außenoberfläche und Innenoberfläche unterbrochen ist. Zur Überbrückung dieser durchgängigen Flächenunterbrechung kann das Anbringteil einen Schließmechanismus umfassen, wie er später beschrieben wird. Die Flächenunterbrechung kann das Einbringen des Gegenstands in einen von der Innenoberfläche umgebenen Raum erleichtern. Aufgrund der offenen Form kann der für die medizinische Anwendung notwendige Teil der anatomischen Struktur, des medizinischen Instruments bzw. Implantats aus dem vom Anbringteil (zumindest teilweise) umschlossenen Teil vorstehen. Die aus dem Anbringteil modellierte Form kann eine Materialdicke mit einer Untergrenze von z.B. 0,5 mm, 1 mm oder 5 mm und/oder einer Obergrenze von z.B. 2 mm, 5 mm oder 1 cm oder 2 cm besitzen. Zudem kann das Anbringteil eine mechanische Schließverbindung (z.B. einen Einrast- bzw. Klickverschluss, einen Klettverschluss, einen Druclcknapfverschluss, eine einem Gurtschloss ähnliche oder gleiche Einrichtung, eine Gewindeverbindung, einen Nagel, eine Schraube, eine Schweißnaht und /oder eine Klebenaht, also eine formschlüssige und/oder kraftschlüssige und/oder reibschlüssige und/oder stoffschlüssige Verbindung, die lösbar und/oder ortsfest gestaltet sein kann) beinhalten, die den kraftschlüssigen und/oder reibschlüssigen und/oder formschlüssigen und/oder stoffschlüssigen Sitz der Marker-Anbringvorrichtung an dem Gegenstand und/oder das Lösen der Marker-Anbringvorrichtung von dem Gegenstand unterstützt. Das Anbringteil umfasst vorzugsweise ein rutschhemmendes Mittel (das z.B. einen Stoff und/oder eine Vorrichtung und/oder ein chemisches Agens sein kann und z.B. durch eine Erhebung aus einer Oberfläche des Anbringteils und eine Stoff- bzw. Materialeigenschaft des Anbringteils gebildet werden kann) an beispielsweise seiner insbesondere dem Gegenstand zugewandten Innenoberfläche. Als seine Innenoberfläche wird die Oberfläche bezeichnet, die im angebrachten Zustand an einer Oberfläche des Gegenstands anliegt bzw. dieser zugewandt ist. Wenigstens eine zusätzliche, insbesondere aus einer seiner Oberflächen herausragende (etwa dorn- oder keilförmige und/oder abgerundete) Erhebung und/oder eine rutschhemmende Oberfläche, die ihre rutschhemmende Eigenschaft z.B. durch eine haft- und/oder gleitreibungsverstärkende Beschichtung (etwa mit einem gummiartigen Stoff bzw. Material, beispielsweise Weich-PVC bzw. Weich-Polyvinylchlorid und/oder vulkanisierter Gummi) oder eine rauheitserhöhende Behandlung (z.B. durch Schleifen oder Sandstrahlen) verliehen bekommt, können einen rutschfesten Sitz des Anbringteils an dem Gegenstand unterstützen. Dadurch kann eine ungewünschte Lageveränderung des Anbringteils relativ zum Gegenstand während der Operation verhindert werden.

Das Anbringteil kann aus mehreren Teilen bestehen. Ein erster Teil des Anbringteils kann aus einem anderen Material als ein zweiter Teil des Anbringteils, der von dem ersten Teil verschieden ist, ausgebildet sein, wobei das Material in dem ersten Teil des Anbringteils insbesondere eine größere spezifische Festigkeit als das Material in dem zweiten Teil des Anbringteils besitzt. Dadurch kann z.B. eine mechanische Beschädigung des Anbringteils während der Benutzung vermieden werden, indem ein stärker belasteter Teil des Anbringteils, der z.B. die mechanische Schließverbindung umfasst, aus härterem und/oder belastbarerem Material oder mit einer größeren Materialdicke als in einem zweiten Teil ausgebildet wird. Ferner kann seine leichtere Anformung unterstützt werden, indem ein für die Verformung vorgesehener Teil des Anbringteils aus einem flexibleren und/oder weniger harten Material als z.B. in einem nicht für eine Anformung vorgesehenen Teil ausgebildet wird.

Die Markervorrichtung, die an der Marker-Anbringvorrichtung befestigt wird, kann aus einem Metall und/oder einem Kunststoff hergestellt sein.

Bei der oben beschriebenen Marker-Anbringvorrichtung kann auf der Oberfläche des Anbringteils, die der Oberfläche des Anbringteils, auf der die Markervorrichtung angebracht ist bzw. die auf die Markervorrichtung zeigt bzw. weist, entgegengesetzt ist, eine Gegenhalterung, die ein Metall oder einen Kunststoff beinhalten, vorgesehen sein. Die Gegenhalterung kann plattenförmig bzw. scheibenförmig, sphärisch oder in Form eines Parallelepipeds oder Polyeders ausgebildet sein und eine Materialdicke mit einer Untergrenze von z.B. 0,5 mm, 1 mm oder 2 mm und/oder einer Obergrenze von z.B. 2 mm, 5 mm oder 1 cm aufweisen. Diejenige Oberfläche der Gegenhalterung, die den festen Sitz der Markervorrichtung an dem Anbringteil unterstützt, kann beispielsweise einen Flächeninhalt mit einer Untergrenze von z.B.5 mm² und/oder einer Obergrenze von z.B. 2 cm² oder 10 cm² besitzen. Ferner kann die Gegenhalterung aus einem flexiblen, elastischen Material (etwa aus einem Gummi oder einem Kunststoff) und/oder einem mechanisch festen Material (z.B. einem Metall nach Art einer handelsüblichen Beilagscheibe) hergestellt sein. Die Gegenhalterung soll ein Verrutschen der Markervorrichtung relativ zum Anbringteil (z. B. durch Wackeln oder unbeabsichtigte Berührung) verhindern.

Die Befestigungsmöglichkeit für die Markervorrichtung an der Marker-Arzbringvarrichtung kann eine mechanische Halteverbindung beinhalten, wobei die mechanische Halteverbindung insbesondere wenigstens eine formschlüssige und/oder kraftschlüssige und/oder reibschlüssige und/oder stoffschlüssige Verbindung beinhaltet. Die mechanische Halteverbindung zum Befestigen der Markervorrichtung an der Marker-Anbringvorrichtung kann in Form einer mechanischen Halteverbindung, z. B. eines Einrastmechanismus', einer Gewindeverbindung, eines Druckknopfes, eines Nagels, einer Schraube, einer Schweißnaht, einer Klebenaht und/oder eines Klettverschlusses ausgestaltet sein. Eine Kombination dieser Halteverbindungsarten kann auch angewandt werden. Vorzugsweise soll die mechanische Halteverbindung formschlüssig und/oder kraftschlüssig und/oder reibschlüssig und/oder stoffschlüssig sowie ortsfest wirken und/oder lösbar sein. Die Befestigungsmöglichkeit kann ferner beispielsweise durch den Rand eines Loches in einer Oberfläche des Anbringteils ausgebildet sein oder dieses umfassen. Die Mechanische Halteverbindung unterstützt den ortsfesten Sitz der Markervorrichtung an der Marker-Anbringvorrichtung und kann die leichte Lösbarkeit der beiden Teile voneinander fördern.

Die Marker-Anbringvorrichtung kann so ausgelegt sein, dass sie an einem medizinischen Instrument und/oder einer anatomischen Körperstruktur und/oder einem Implantat anbringbar ist. Hierfür müssen jeweils passende Materialkombinationen zur Gewährleistung eines kraftschlüssigen und/oder reibschlüssigen und/oder formschlüssigen Sitzes gewählt werden. In letzteren beiden Fällen muss auch an die physiologische Verträglichkeit der Materialien der Marker-Anbringvorrichtung gedacht werden.

Die Marker-Anbringvorrichtung kann eine Markervorrichtung beinhalten, die wiederum einen Referenzstern und/oder wenigstens ein Markerelement beinhaltet. Diese Markervorrichtung kann mittels o.g. Befestigungsmöglichkeit an dem Anbringteil der Marker-Anbringvorrichtung befestigt werden.

Die Marker-Anbringvorrichtung und ein medizinisches Gerät und/oder ein Implantat können ein System bilden, das dem Anwender als Paket zur Verfügung steht.

Ferner können die Marker-Anbringvorrichtung und eine Markervorrichtung ein System bilden, das dem Anwender als Paket zur Verfügung steht.

Die Verwendung einer Marker-Anbringvorrichtung wie oben beschrieben kann erfolgen, indem die Anbringvorrichtung an einem Gegenstand, insbesondere an einem medizinischen Instrument und/oder einer anatomischen Körperstruktur und/oder einem Implantat, angebracht wird.

Ein Verfahren zum Befestigen der oben beschriebenen Marker-Anbringvorrichtung an einem Gegenstand, insbesondere an einem medizinischen Instrument und/oder einer anatomischen Körperstruktur und/oder einem Implantat, kann folgenden Ablauf beinhalten: Zuerst wird dem Material des Anbringteils Energie zugeführt, um es beispielsweise zu erwärmen. Hierbei wirkt Energie aus z.B. mechanischen Schwingungen bzw. Wellen und/oder thermischer Energie und/oder elektromagnetischer Strahlung und/oder eine chemische Reaktion und/oder elektrischen Strom (also Energie aus physikalischen bzw. chemischen Verfahren) auf das Material des Anbringteils ein, um das Material des Anbringteils leichter als vor der Energieeinwirkung, plastisch verformbarer, d.h. leichter plastisch verformbar, zu machen. Insbesondere kann das Anbringteil vor Energieeinwirkung nicht plastisch verformbar, also hart sein. Das Anbringteil wird dadurch mit von einer menschlichen Hand ohne weitere Hilfsmittel erzielbaren Kräften plastisch verformbarer, also z.B. mit bloßen Händen mit Kräften, deren Untergrenze bei etwa 0,1 N, 1 N oder 10 N und/oder deren Obergrenze bei etwa 10 N, 100 N oder 1000 N liegt. Dann wird das Anbringteil an die Struktur (d.h. äußere Form) des Gegenstandes angeformt. Schließlich kann das Material des Anbringteils aushärten, d.h. härter gemacht werden. Auch folgender Verlauf ist möglich: Das Material des Anbringteils wird in einer Rohform an den Anwender weitergegeben, die das Material in einem plastisch verformbaren Zustand (d.h. einem Verformungszustand) beinhaltet. In diesem Zustand ist das Material des Anbringteils mit von einer menschlichen Hand ohne weitere Hilfsmittel erzielbaren Kräften, also z.B. mit bloßen Händen mit Kräften, deren Untergrenze bei etwa 0,1 N, 1 N oder 10 N und deren Obergrenze bei etwa 10 N, 100 N oder 1000 N liegt, verformbar. Dann wird das Anbringteil an die Struktur (d.h. äußere Form) des Gegenstandes angeformt. Schließlich wird dem Material des Anbringteils Energie zugeführt, um es beispielsweise zu erwärmen. Hierbei wirken beispielsweise mechanischen Schwingungen bzw. Wellen und/oder thermische Energie und/oder elektromagnetische Strahlung und/oder eine chemische Reaktion und/oder elektrischer Strom (also Energie aus physikalischen bzw. chemischen Verfahren) auf das Material des Anbringteils ein, um es auszuhärten, d.h. in einen mechanisch festeren, insbesondere härteren und belastbareren Zustand (den Verwendungszustand) überzuführen. Die Aussage bezüglich der obigen Verfahren, dass das Material des Anbringteils durch Energieeinwirkung plastisch verformbar wird, beinhaltet, dass das Material des Anbringteils im Verformungszustand plastisch verformbarer (d.h. leichter bzw. mit geringerem Kraftaufwand verformbar) wird als vor der Energieeinwirkung. Im Verwendungszustand kann das Material des Anbringteils insbesondere eine höhere Zugfestigkeit und/oder Druckfestigkeit und/oder Scherfestigkeit erlangen als im Zustand der Rohform und/oder im Verformungszustand. Das Material des Anbringteils kann im Verwendungszustand auch mechanisch härter als im Zustand der Rohrform und/oder im Verformungszustand vorliegen.

Ein Verfahren zum Entfernen der oben beschriebenen Marker-Anbringvorrichtung von einem Gegenstand, insbesondere von einem medizinischen Instrument und/oder einer anatomischen Körperstruktur und/oder einem Implantat kann folgenden Ablauf beinhalten: Zuerst wird dem Material des Anbringteils Energie zugeführt, um es beispielsweise zu erwärmen. Hierbei wirken beispielsweise mechanische Schwingungen bzw. Wellen und/oder thermische Energie und/oder elektromagnetische Strahlung und/oder eine chemische Reaktion und/oder elektrischer Strom (d.h. Energie aus physikalischen bzw. chemischen Verfahren) auf das Material des Anbringteils ein, um das Anbringteil leichter verformbar als vor der Energieeinwirkung zu machen. Das Material des Anbringteils wird mit von einer menschlichen Hand ohne weitere Hilfsmittel erzielbaren Kräften, also z.B. mit bloßen Händen mit Kräften, deren Untergrenze bei etwa 0,1 N, 1 N oder 10 N und/oder deren Obergrenze bei etwa 10 N, 100 N oder 1000 N liegt, plastisch verformbar. Schließlich kann das Anbringteil von dem Gegenstand z.B. mit einem Auftrennmittel (z.B. einer Schere oder einem Messer oder einer Zange) räumlich entfernt werden.

Weitere Vorteile und Merkmale der Erfindung werden bei der folgenden detaillierten Beschreibung offenbart. Merkmale unterschiedlicher Ausführungsformen können untereinander kombiniert werden.

Vorteilhaft gewährleistet das Anbringteil einen festen Sitz der Markervorrichtung am Gegenstand während aller Arbeitsphasen der Operation. Ferner sollte es vorteilhaft Eigenschaften besitzen, die seine einwandfreie Hygiene gewährleisten, z. B. die Beständigkeit gegen üblicherweise verwendete chemische Desinfektionsmittel bzw. Reinigungsverfahren (z. B. durch Erhitzen). Alternativ kann das Anbringteil als Einwegartikel hergestellt werden, der nach der Verwendung entsorgt wird. Beispielsweise kann es aus einem Kunststoff und/oder einem metallischen Blech hergestellt werden, wobei diese Materialien in übliche Entsorgungssysteme problemlos integriert werden können.

Ferner sollte das Material, aus dem das Anbringteil hergestellt ist, den chemischen und mechanischen Belastungen während einer Operation standhalten können. Dies kann durch Verwendung eines Kunststoffes geschehen, der beispielsweise aramidhaltige Fasern beinhaltet.

Vorteilhaft kann das Anbringteil universell an verschiedene Oberflächen angepasst werden, damit man es an diverse medizinische Instrumente anbringen kann. Vorteilhaft wird das Anbringteil an den Benutzer in einer Rohform geliefert, die der Benutzer dann an die Oberfläche des betreffenden Instrumentes anformen kann, wobei das Anbringteil von einem Verformungs- in einen Verwendungszustand gelangt. Vorteilhaft ist das Anbringteil mit von einer menschlichen Hand ohne weitere Hilfsmittel erzielbaren Kräften, also z.B. mit bloßen Händen mit Kräften, deren Untergrenze bei etwa 0,1 N, 1 N oder 10 N und deren Obergrenze bei etwa 10 N, 100 N oder 1000 N liegt, verformbar, um eine leichte Handhabbarkeit zu gewährleisten.

Um die Verwendbarkeit des Anbringteils zu erweitern, kann das Anbringteil derart gestaltet werden, dass man es nicht nur an medizinischen Instrumenten sondern auch an anatomischen Körperstrukturen anbringen kann. Hierbei sind zusätzlich zu den oben geforderten Eigenschaften des erfindungsgemäßen Gegenstandes die besonders leichte Handhabbarkeit, Verträglichkeit durch den menschlichen Körper sowie die Vermeidung von Verletzung bei Patient und Anwender zu beachten. Dies kann durch die Verwendung bioverträglicher bzw. physiologisch verträglicher Polymere im Material des Anbringteils wie etwa Silikonpolymere erreicht werden.

Vorteilhaft kann das Anbringteil derart gestaltet sein, dass es vom menschlichen Körper resorbiert werden kann, so dass eine Entfernung nach dem Ende der Operation nicht notwendig ist. Dies kann durch die Verwendung bioverträglicher resorbierbarer Polymere im Material des Anbringteils wie etwa Polylaktid, Polyglykolid (allgemein Polymere auf Basis von Milchsäure und/oder Glykolsäure) erreicht werden.

Figur 1 zeigt den Auformprozess einer erfindungsgemäßen Marker-Anbringvorrichtung mit einem schlauchförmigen Anbringteil und einer Befestigungsmöglichkeit für eine Markervorrichtung an ein medizinisches Instrument.

Figur 2 zeigt die erfindungsgemäße Marker-Anbringvorrichtung in Form eines Riemens mit einer Schließvorrichtung in Form eines Einrastverschlusses.

Figur 3 zeigt die erfindungsgemäße Marker-Anbringvorrichtung mit einem Anbringteil in Form eines Riemens mit einer Schließvorrichtung in Form eines Einrastverschlusses und mit einer rutschhemmenden Beschichtung auf der dem Gegenstand zugewandten Oberfläche des Anbringteils.

Figur 4 zeigt die erfindungsgemäße Marker-Anbringvorrichtung mit einem Anbringteil in Form eines Riemens mit einer Schließvorrichtung in Form eines Einrastverschlusses und einer mechanischen Halteverbindung in Form einer Fixierschraube.

Figur 5 zeigt die erfindungsgemäße Marker-Anbringvorrichtung mit einem zumindest teilweise netzförmig ausgebildeten Anbringteil und einer Schließvorrichtung in Form eines Einrastverschlusses.

Figuren 6a und 6b zeigen die erfindungsgemäße Marker-Anbringvorrichtung mit einem Anbringteil und einer Schließvorrichtung in Form eines Einrastverschlusses sowie verschiedene Ausgestaltungen eines Rutschverhinderungsmittels zur Unterstützung eines rutschfesten Sitzes des Anbringteils.

Figur 7 zeigt die erfindungsgemäße Marker-Anbringvorrichtung, und wie sie an einer anatomischen Körperstruktur oder einem Implantat beispielsweise befestigt werden kann.

Figur 8 zeigt die erfindungsgemäße Marker-Anbringvorrichtung mit einer an ihr angebrachten Markervorrichtung.

Figur 9 zeigt die erfindungsgemäße Marker-Anbringvorrichtung mit einem einzelnen an ihr angebrachten Markerelement.

Figur 10 zeigt die erfindungsgemäße Marker-Anbringvorrichtung mit einer Gegenhalterung zur Stabilisierung einer mit der Marker-Anbringvorrichtung verbundenen Markervornchtung.

Figur 11 zeigt einen ersten Verfahrensablauf zum Befestigen eines Anbringteils einer Marker-Anbringvorrichtung an einem Gegenstand.

Figur 12 zeigt einen zweiten Verfahrensablauf zum Befestigen eines Anbringteils einer Marker-Anbringvorrichtung an einem Gegenstand.

Figur 13 zeigt einen dritten Verfahrensablauf zum Befestigen eines Anbringteils einer Marker-Anbringvorrichtung an einem Gegenstand.

Figur 14 zeigt einen vierten Verfahrensablauf zum Befestigen eines Anbringteils einer Marker-Anbringvorrichtung an einem Gegenstand.

Figur 15 zeigt einen Verfahrensablauf zum Entfernen eines Anbringteils einer Marker-Anbringvorrichtung von einem Gegenstand.

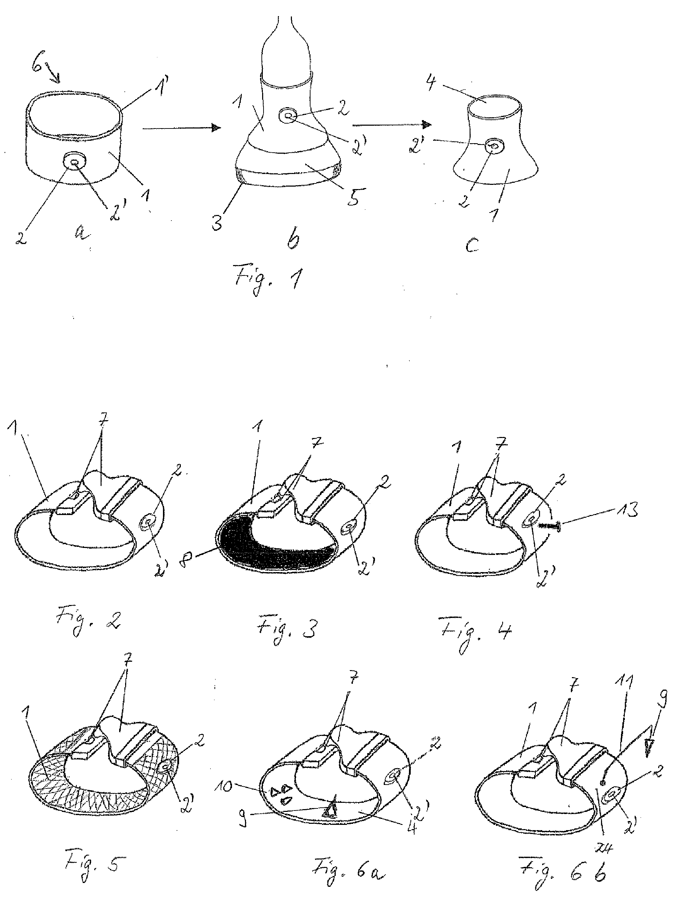

Das Anbringteil 1 kann beispielsweise in einer Rohform an den Benutzer geliefert werden, die zur Anformung and den Gegenstand 3, 14 vorgesehen ist. Aus der Rohform entsteht eine Benutzungsform (bei der sich das Anbringteil 1 in einem Verwendungszustand befindet), die den Halt des Anbringteils 1 an dem Gegenstand 3, 14 bewirkt. Diese Rohrform kann vorteilhaft schlauchförmig wie in Figur 1a ausgestaltet sein. Sie kann z.B. aber auch eine Rohrform mit einer offenen Fläche, wie einem Toroid oder einem Riemen (wie in Figuren 2-4) oder einem quaderförmigen und/oder sphärischen bzw. ellipsoidischen Materialklumpen haben. In der Rohform kann in einer Ausführungsform der Erfindung das Material des Anbringteils 1 in einem mechanisch festen Zustand oder auch in einem verformbaren Zustand (d.h. Verformungszustand) vorliegen.

Figur 1 zeigt, wie ein erfindungsgemäßes, schlauchförmiges Anbringteil 1 mit einer Befestigungsmöglichkeit 2 für eine Markervorrichtung über einen Gegenstand, hier ein konusförmiges medizinisches Instrument 3 oder ein anatomischer Körperteil bzw. ein Implantat 14, gestülpt wird (vgl. Figuren 1, 7 und 11[Bezugszeichen S1]). Das Anbringteil 1 kann insbesondere einstückig sein, aber auch aus mehreren Stücken (d.h. aus wenigstens zwei Stücken) bestehen. Anschließend wird das Anbringteil 1 durch Energieeinwirkung in einen plastisch verformbaren Zustand (Vcrformungszustand) gebracht (S2); dann kann das Anbringteil 1 so verformt werden (S3), dass seine auf die Oberfläche 5 des Gegenstands 3 weisende Oberfläche 4 an die Oberfläche 5 des Gegenstands 3 angepasst (S4) werden kann. Hierdurch wird ein formschlüssige Sitz des Anbringteils 1 an dem Gegenstand 3 hergestellt. Das Anbringteil 1 kann in der Benutzungsform aber auch eine offene Form haben, bei der die zwei Außenkanten unterbrochen sind. Zur Überbrückung dieser durchgängigen Flächenunterbrechung kann das Anbringteil einen Schließmechanismus umfassen, wie er später beschrieben wird. Die Flächenunterbrechung kann das Einbringen des Gegenstands in einen von der Innenoberfläche umgebenen Raum erleichtern. Die Form gibt dem Anbringteil 1 die Form eines Clips mit einem formschlüssigen und/oder kraftschlüssigen und/oder reibschlüssigen Halt an dem Gegenstand 3 gibt. Schließlich muss das Material des Anbringteils 1 aushärten, d.h. härter bzw. fester gemacht werden (S5), was z.B. durch Trocknung an der Umgebungsluft oder unterstützende Behandlung mit angewärmter oder abgekühlter Luft aus einem Fön bzw. Ventilator geschehen kann.

Die Verformbarkeit des Materials des Anbringteils 1 kann beispielsweise dadurch bewirkt werden, dass das Anbringteil 1 aus einem Material hergestellt wird, das unter der Einwirkung akustischer Schwingungen und Wellen und/oder mechanischer Scherung bzw. Scherschwingungen und -wellen plastisch verformbar wird, also in den Verformurzgszustand gebracht wird. Hierzu muss das Material des Anbringteils vor der Anpassung an eine gegebene Oberfläche eines Gegenstands mit den entsprechenden Schwingungen bzw. Wellen mechanisch beansprucht werden. Für diese Vorgehensweise in Frage kommende Materialien des Anbringteils 1 sind Thermoplaste wie z. B. Polyamide und/oder Polycarbonate und/oder Polyethylentherephthalat und/oder Polyethylen und/oder Polypropylen und/oder Polystyrol und/oder Polyvinylchlorid.

Die Verformbarkeit des Materials des Anbringteils 1 kann beispielsweise dadurch bewirkt werden, dass das Anbringteil 1 ein Material (wie etwa Polyethylen und/oder Polycarbonat und/oder Polyamide und/oder Polyester und/oder Polyacrylate) beinhaltet, das unter der Einwirkung von Ultra- und/oder Infraschallwellen plastisch verformbar wird. Hierzu muss das Material des Anbringteils 1 vor der Anpassung an eine gegebene Oberfläche 5 eines Gegenstands 3 mit Ultra- und Infraschallwellen geeigneter Intensität und über geeignete Dauer bestrahlt werden. Weitere für dieses Verfahren in Frage kommende Materialien des Anbringteils 1 sind Thermoplaste wie z. B. Polyamide und/oder Polycarbonate und/oder Polyethylentherephthalat und/oder Polyethylen und/oder Polypropylen und/oder Polystyrol und/oder Polyvinylchlorid.

In einer Ausführungsform der Erfindung kann die Verformbarkeit des Materials des Anbringteil 1 dadurch bewirkt werden, dass das Anbringteil ein Material beinhaltet, das unter der Einwirkung von thermischer Energie plastisch verformbar wird. Hierzu muss vor der Anformung des Anbringteils 1 an den Gegenstand 3 das Material des Anbringteils 1 dem Einfluss thermischer Energie ausgesetzt, d.h. mit einer geeigneten Energiemenge auf eine geeignete Temperatur erwärmt werden. Anschließend kann eine plastische Verformung und Anpassung des Anbringteils 1 an eine gegebene Oberfläche 5 erfolgen. Bei diesen Stoffen kann eine Erwärmung auf eine Temperatur, die in einem Bereich liegt, der eine untere Grenze von 30°C oder 40°C oder 50°C und eine obere Grenze von 190°C oder 200°C oder 210°C besitzt, und/oder die beispielsweise bei 60°C oder 100°C oder 150°C liegt, ausreichen, um die plastische Verformbarkeit des Materials des Anbringteils 1 zu erzielen.

In einer weiteren Ausführungsform der Erfindung kann die Verformbarkeit des Materials des Anbringteils 1 dadurch bewirkt werden, dass das Anbringteil 1 ein Material beinhaltet, das unter der Einwirkung von Infrarotstrahlung plastisch verformbar wird. Hierzu muss vor der Anpassung des Anbringteils 1 an eine gegebene Oberfläche 5 das Material des Anbringteils 1 dem Einfluss von Infrarotstrahlung ausgesetzt werden. Dies kann z.B. durch Bestrahlung mit einer Lichtquelle, die elektromagnetische Strahlung im Infrarotbereich aussendet, erfolgen. Anschließend kann das Anbringteil 1 an eine gegebene Oberfläche 5 eines Gegenstandes 3 angeformt werden. Für diese Vorgehensweise in Frage kommende Materialien des Anbringteils 1 sind Thermoplaste wie z. B. Polyamide und/oder Polycarbonate und/oder Polyethylentherephthalat und/oder Polyethylen und/oder Polypropylen und/oder Polystyrol und/oder Polyvinylchlorid.

In einer weiteren Ausführungsform der Erfindung kann die Verformbarkeit des Materials des Anbringteils 1 dadurch bewirkt werden, dass das Anbringteil 1 ein Material beinhaltet, das unter der Einwirkung von Mikrowellenstrahlen plastisch verformbar wird. Hierzu muss vor Anpassung des Anbringteils 1 an eine gegebene Oberfläche 5 das Material des Anbringteils 1 dem Einfluss von Mikrowellenstrahlung ausgesetzt werden. Dies kann z.B. dadurch geschehen, dass das Material des Anbringteils in einen handelsüblichen Mikrowellenofen eingebracht wird und darin mit Mikrowellenbestrahlung bestrahlt wird. Anschließend kann das Anbringteil 1 an eine gegebene Oberfläche 5 angepasst werden. Für diese Vorgehensweise in Frage kommende Materialien des Anbringteils 1 sind Thermoplaste wie z. B. Polyamide und/oder Polycarbonate und/oder Polyethylentherephthalat und/oder Polyethylen und/oder Polypropylen und/oder Polystyrol und/oder Polyvinylchlorid.

In einer weiteren Ausführungsform der Erfindung kann die Verformbarkeit des Materials des Anbringteils 1 dadurch bewirkt werden, dass das Anbringteil 1 ein Material beinhaltet, das unter der Einwirkung von ultravioletter Strahlung plastisch verformbar wird. Hierzu kann das Material des Anbringteils 1 z.B. mit einer Lichtquelle bestrahlt werden, die elektromagnetische Strahlung im ultravioletten Spektralbereich emittiert. Anschließend kann das Anbringteil 1 verformt und an eine gegebene Oberfläche 5 angepasst werden.

Beispielsweise kann die Verformbarkeit des Materials des Anbringteils 1 auch dadurch bewirkt werden, dass das Anbringteil ein Material beinhaltet, das unter der Einwirkung von sichtbarem Licht plastisch verformbar wird. Hierzu kann das Material z.B. mit einer Lichtquelle bestrahlt werden, die elektromagnetische Strahlung im sichtbaren Spektralbereich emittiert. Anschließend kann das Anbringteil verformt und an eine gegebene Oberfläche angepasst werden. Für diese Vorgehensweise in Frage kommende Materialien des Anbringteils 1 sind Thermoplaste wie z. B. Polyamide und/oder Polycarbonate und/oder Polyethylentherephthalat und/oder Polyethylen und/oder Polypropylen und/oder Polystyrol und/oder Polyvinylchlorid.

In einer anderen Ausführungsform kann die Verformbarkeit des Materials des Anbringteils 1 dadurch bewirkt werden, dass das Anbringteil 1 ein Material beinhaltet, das unter der Einwirkung bestimmter chemischer Reaktionen plastisch verformbar wird. Hierzu kann das Material des Anbringteils 1 beispielsweise einer äußeren Behandlung mit einer chemischen Verbindung ausgesetzt werden, die etwa durch ein Bad in einer flüssigen oder gasförmigen chemischen Verbindung durchgeführt wird. Alternativ kann das Material des Anbringteils 1 derart gestaltet sein, dass es aus unterschiedlichen Komponenten aufgebaut ist, die bestimmte chemische Verbindungen enthalten, die in Art eines festen Zweikomponentenklebers (z.B.

Methylmethacrylat-Klebstoffs) miteinander durch z.B. Kneten vermengt werden können und so eine chemische Reaktion eingehen, die das Material des Anbringteils plastisch verformbar werden lässt. Dies kann beispielhaft auch durch eine exotherme chemische Reaktion derart bewirkt werden, dass die bei der Reaktion frei werdende thermische Energie das Material des Anbringteils 1 plastisch verformbar werden lässt.

Die Aussage bezüglich der obigen Ausführungsformen, dass das Material des Anbringteils 1 durch Extergieeinwirkung plastisch verformbar wird, beinhaltet, dass das Material des Anbringteils 1 im Verformungszustand plastisch verformbarer (d.h. leichter bzw. mit geringerem Kraftaufwand verformbar) wird als vor der Energieeinwirkung. Im Verwendungszustand kann das Material des Anbringteils 1 insbesondere eine höhere Zugfestigkeit und/oder Druckfestigkeit und/oder Scherfestigkeit erlangen als im Zustand der Rohform und/oder im Verformungszustand. Das Material des Anbringteils 1 kann im Verwendungszustand auch mechanisch härter als im Zustand der Rohform und/oder im Verformungszustand vorliegen.

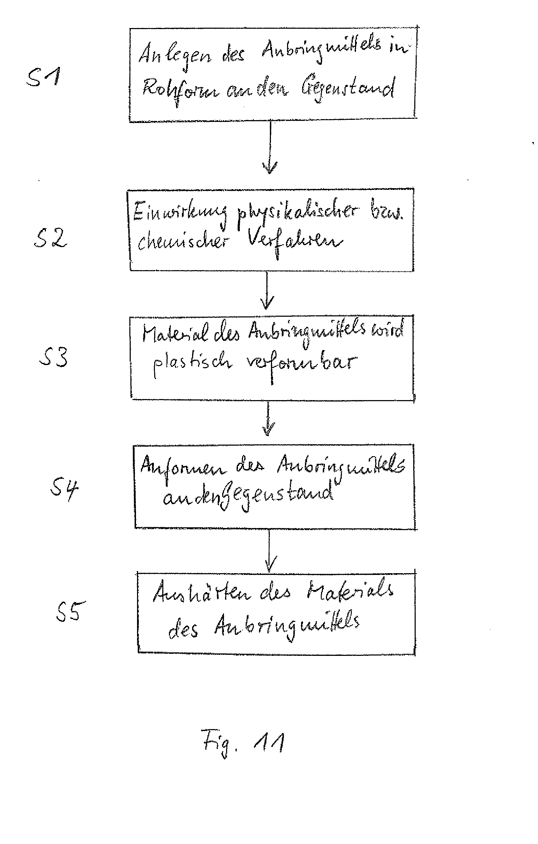

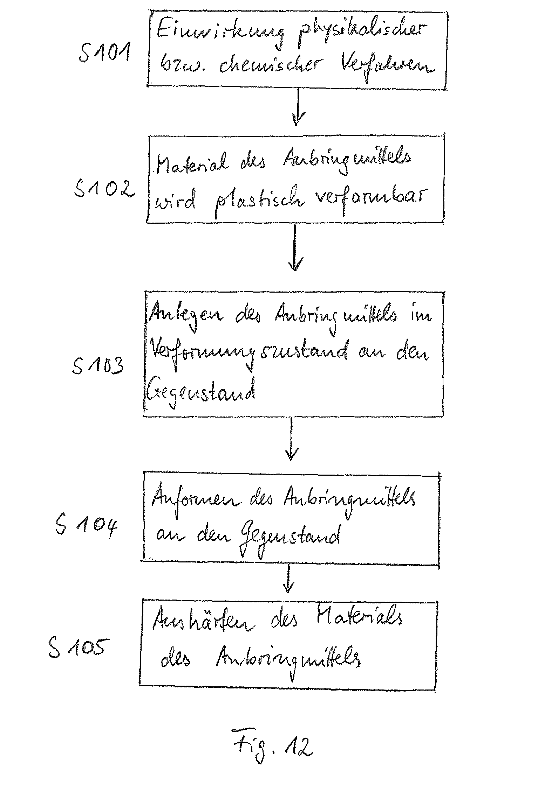

Gegebenenfalls kann das Anbringteil 1 wie in Figur 11 gezeigt in seiner Rohform an die gegebene Oberfläche 5 eines Gegenstandes 3 angelegt werden (S1), um dann erst die plastische Verformbarkeit (d.h. einem Verformungszustand) mit einem der oben genannten Verfahren herzustellen (S2, S3) und anschließend die gewünschte Anformung an die Oberfläche 5 durchzuführen (S4). Hierzu muss jedoch die Oberfläche bzw. der Gegenstand dergestalt sein, dass sie bzw. er den physikalischen und/oder chemischen Einflüssen des gewählten Verfahrens standhält. Wahlweise kann der Vorgang wie in Figur 12 gezeigt auch so erfolgen, dass das Anbringteil 1 räumlich getrennt von der Oberfläche 5 des Gegenstands 3 nach einem der oben genannten Verfahren (S101) in plastische Verformbarkeit (d.h. in einen Verformungszustand) versetzt (S102) und dann erst an den Gegenstand 3 angelegt (S103) und angeformt (S104) wird. Diese Verfahrensweise hat den Vorteil, dass eine Oberfläche 5 des Gegenstands 3 (insbesondere eines medizinischen Instruments bzw. einer anatomischen Struktur), die gegenüber dem Standardgebrauch erhöhten chemischen Belastungen gemäß dem Verfahren der Figur 11 standhält, unbeeinträchtigt bleibt.
Nach dem Anformen (S4, S104) des Anbringteils 1 an die gegebene Oberfläche 5 wird das Material des Anbringteils vorzugsweise ausgehärtet bzw. mechanisch fester gemacht (S5, S105), d.h. in eine Benutzungsform bzw. einen Verwendungszustand versetzt, um die für einen stabilen Sitz der Marker-Anbringvorrichtung 6 an dem Gegenstand 3, 14 erforderliche mechanische Festigkeit zu erlangen.

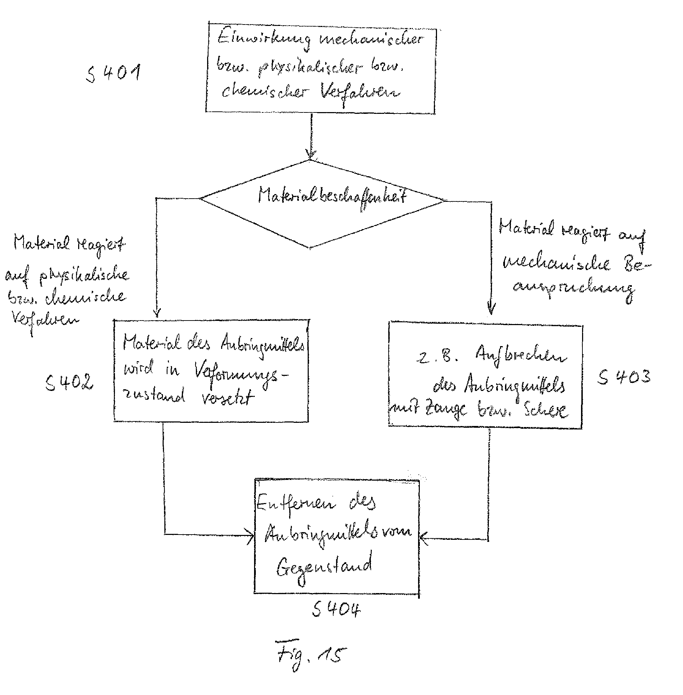

Das in Figur 15 gezeigte Entfernen (S404) des Anbringteils 1 von dem Gegenstand 3, 14 kann gegebenenfalls auf mechanischem Wege, z.B. mit Hilfe einer Auftrennvorrichtung, z. B. einer Schere oder einem Messer oder einer Zange (S403) erfolgen. Insbesondere wird wenigstens eines der physikalisch-chemischen Verfahren (S2, S101, S401) zum Entfernen des Anbringteils 1 von dem Gegenstand 3, 14 verwendet, die dazu verwendet werden können, um das Material des Anbringteils 1 aus der festen Rohform in einen Verfozxnungszustatxd (S3, S102, S402) zu versetzen, der dann ein einfaches Ablösen (404) des Anbringteils 1 von dem Gegenstand 3, 14 erlaubt.

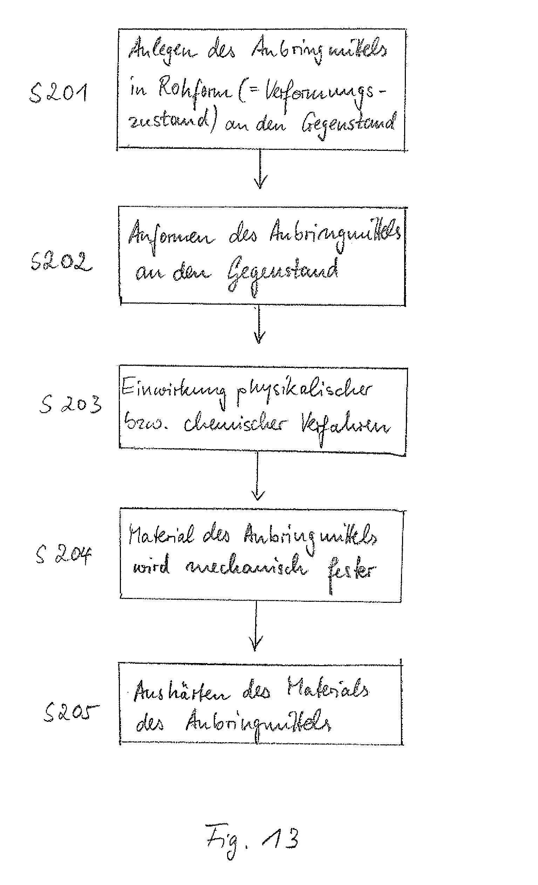

Es kann aber auch - wie in Figur 13 gezeigt - in einer wiederum weiteren Ausführungsform das Anbringteil 1 bereits vor dem Überstülpen über das medizinische Instrument 3 als Rohform in einem Verformungszustand (S201) vorliegen und über das medizinische Instrument 3 gestülpt und angeformt werden, ohne dass der Anwender die Verformbarkeit erst herstellen muss (vgl. Verfahrensablauf in Figur 13). Das Aushärten bzw. Festigen (S204, S205) kann dann beispielsweise durch Lufttrocknung erfolgen: Dazu muss das Material nur an der Umgebungsluft bei Raumtemperatur trocknen, vorzugsweise kann aber auch der Aushärtungsprozesses durch einen Luftfön mit unter Umständen gegenüber der Umgebungsluft angewärmter oder abgekühlter Luft unterstützt werden, um dem Material die gewünschte Festigkeit zu verleihen.

Das Aushärten bzw. Festigen (S204, S205) des Materials des Anbringteils 1 kann beispielsweise dadurch bewirkt werden, dass das Anbringteil 1 ein Material beinhaltet, das unter der Einwirkung (S203) akustischer Schwingungen und Wellen und/oder mechanischer Scherung bzw. Scherschwingungen und -wellen mechanisch fest wird. Hierzu muss das Material des Anbringteils vor der Anpassung an eine gegebene Oberfläche eines Gegenstands mit den entsprechenden Schwingungen bzw. Wellen mechanisch beansprucht werden.

Das Aushärten bzw. Festigen (S204, S205) des Materials des Anbringteils 1 kann beispielsweise dadurch bewirkt werden, dass das Anbringteil 1 ein Material beinhaltet, das unter der Einwirkung (203) von Ultra- und/oder Infraschallwellen mechanisch fest wird. Hierzu muss das Material des Anbringteils 1 vor der Anpassung an eine gegebene Oberfläche 5 eines Gegenstands 3 mit Ultra- und Infraschallwellen geeigneter Intensität und über geeignete Dauer bestrahlt werden.

In einer Ausführungsform der Erfindung kann das Aushärten bzw. Festigen (S204, S205) des Materials des Anbringteils 1 dadurch bewirkt werden, dass das Anbringteil 1 ein Material beinhaltet, das unter der Einwirkung (S203) von thermischer Energie mechanisch fest wird. Hierzu muss vor der Anformung des Anbringteils 1 an den Gegenstand 3 das Material des Anbringteils dem Einfluss thermischer Energie ausgesetzt, d.h. mit einer geeigneten Energiemenge auf eine geeignete Temperatur erwärmt oder abgekühlt werden.

In einer weiteren Ausführungsform der Erfindung kann das Aushärten bzw. Festigen (S204, S205) des Materials des Anbringteils 1 dadurch bewirkt werden, dass das Anbringteil 1 ein Material beinhaltet, das unter der Einwirkung (S203) von Infrarotstrahlung plastisch verformbar wird. Hierzu muss vor der Anpassung des Anbringteils 1 an eine gegebene Oberfläche 5 das Material des Anbringteils 1 dem Einfluss von Infrarotstrahlung ausgesetzt werden. Dies kann z.B. durch Bestrahlung mit einer Lichtquelle, die elektromagnetische Strahlung im Infrarotbereich aussendet, erfolgen. Anschließend kann das Anbringteil 1 an eine gegebene Oberfläche 5 eines Gegenstandes 3 angeformt werden.

In einer weiteren Ausführungsform der Erfindung kann das Aushärten bzw. Festigen (S204, S205) des Materials des Anbringteils 1 dadurch bewirkt werden, dass das Anbringteil 1 ein Material beinhaltet, das unter der Einwirkung (203) von Mikrowellenstrahlen plastisch verformbar wird. Hierzu muss vor Anpassung des Anbringteils 1 an eine gegebene Oberfläche 5 das Material des Anbringteils 1 dem Einfluss von Mikrowellenstrahlung ausgesetzt werden. Dies kann z.B. dadurch geschehen, dass das Material des Anbringteils in einen handelsüblichen Mikrowellenofen eingebracht wird und darin mit Mikrowellenbestrahlung bestrahlt wird. Anschließend kann das Anbringteil 1 an eine gegebene Oberfläche 5 angepasst werden.

In einer weiteren Ausführungsform der Erfindung kann das Aushärten bzw. Festigen (S204, S205) des Materials des Anbringteils 1 dadurch bewirkt werden, dass das Anbringteil 1 ein Material beinhaltet, das unter der Einwirkung (S203) von ultravioletter Strahlung plastisch verformbar wird. Hierzu kann das Material des Anbringteils 1 z.B. mit einer Lichtquelle bestrahlt werden, die elektromagnetische Strahlung im ultravioletten Spektralbereich emittiert. Anschließend kann das Anbringteil 1 verformt und an eine gegebene Oberfläche 5 angepasst werden.

Vorteilhaft kann das Aushärten bzw. Festigen (S204, S205) des Materials des Anbringteils 1 dadurch bewirkt werden, dass das Anbringteil 1 ein Material beinhaltet, das unter der Einwirkung (S203) von sichtbarem Licht plastisch verformbar wird. Hierzu kann das Material z.B. mit einer Lichtquelle bestrahlt werden, die elektromagnetische Strahlung im sichtbaren Spektralbereich emittiert. Anschließend kann das Anbringteil verformt und an eine gegebene Oberfläche angepasst werden.

Vorteilhaft kann das Aushärten bzw. Festigen (S204, S205) des Materials des Anbringteils 1 dadurch bewirkt werden, dass das Anbringteil 1 ein Material beinhaltet, das unter der Einwirkung (S203) bestimmter chemischer Reaktionen fest wird. Hierzu kann das Material des Anbringteils 1 beispielsweise einer äußeren Behandlung mit einer chemischen Verbindung ausgesetzt werden, die etwa durch ein Bad in einer flüssigen oder gasförmigen chemischen Verbindung durchgeführt wird. Alternativ kann das Material des Anbringteils 1 derart gestaltet sein, dass es unterschiedlichen Komponenten aufgebaut ist, die bestimmte chemische Verbindungen enthalten, die in Art eines mit menschlichen Kräften plastisch verformbaren Zweikomponentenklebers (z.B. Methylmethaerylat-Klebstoffs) miteinander z.B. durch Kneten vermengt werden können und so eine chemische Reaktion eingehen, die das Material des Anbringteils mechanisch fest werden lässt. Dies kann beispielhaft auch durch eine exotherme und/oder endotherme chemische Reaktion derart bewirkt werden, dass die bei der Reaktion frei werdende oder der Umgebung entzogene thermische Energie das Material des Anbringteils 1 mechanisch fester (d.h. fester als vor der Reaktion) werden lässt.

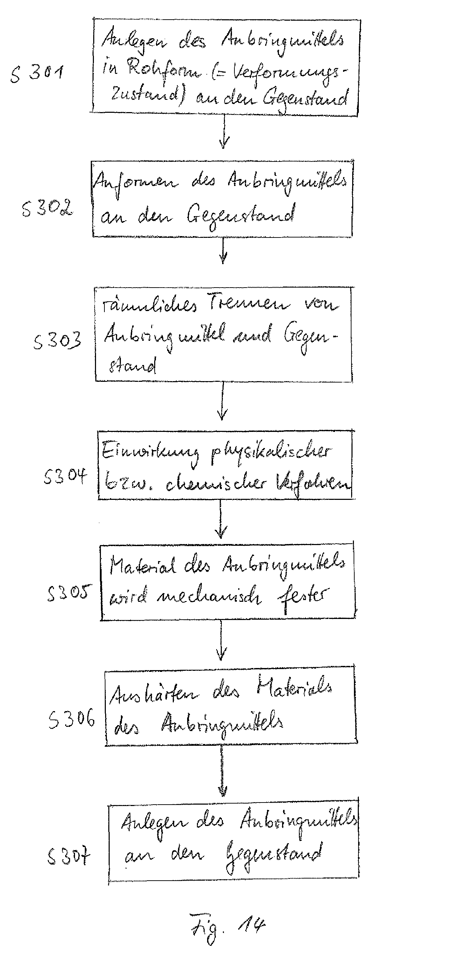

In dieser Ausführungsform kann das Anbringteil 1 - wie in Figur 14 dargestellt - in seiner Rohform an die gegebene Oberfläche 5 eines Gegenstandes 3, 14 angelegt werden (S201) und anschließend die gewünschte Anformung (S202) an die Oberfläche 5 durchgeführt werden, um dann erst die mechanische Festigkeit mit einem der oben genannten Verfahren herzustellen. Hierzu muss jedoch die Oberfläche 5 bzw. der Gegenstand 3, 14 dergestalt sein, dass sie bzw. er den physikalischen und/oder chemischen Einflüssen des gewählten Verfahrens standhält. Wahlweise kann der Vorgang auch so erfolgen, dass das Anbringteil 1 erst an den Gegenstand 3, 14 angelegt (S301) und verformt (S302) wird, anschließend räumlich getrennt (S303) von der Oberfläche 5 des Gegenstands 3 nach einem der oben genannten Verfahren (S304) in einen festen Zustand (S305) versetzt und nach dem Aushärten bzw. Festigen (S306) wieder and den Gegenstand 3, 14 angelegt (S307) wird. Diese Verfahrensweise hat den Vorteil, dass eine Oberfläche 5 des Gegenstands 3 (insbesondere eines medizinischen Instruments bzw. einer anatomischen Struktur), die gegenüber dem Standardgebrauch erhöhten chemischen Belastungen gemäß dem Verfahren der Figur 11 standhält, unbeeinträchtigt bleibt.

Das in Figur 15 gezeigte Entfernen (S404) des Anbringteils 1 von dem Gegenstand 3, 14 kann gegebenenfalls auf mechanischem Wege, z.B. mit Hilfe eines Auftrennmittels, etwa in Form einer Schere oder einer Zange (S403) erfolgen. Vorzugsweise wird wenigstens eines der physikalisch-chemischen Verfahren (S2, S101, S401) angewandt, die zum Aushärten bzw. Festigen (S204, S205, S305, S306) verwendet werden können, um das Material des Anbringteils 1 wieder in einen Verformungszustand (d.h. in seine Rohform) zu versetzen, der dann ein einfaches manuelles Ablösen (S404) des Anbringteils 1 von dem Gegenstand 3, 14 erlaubt.

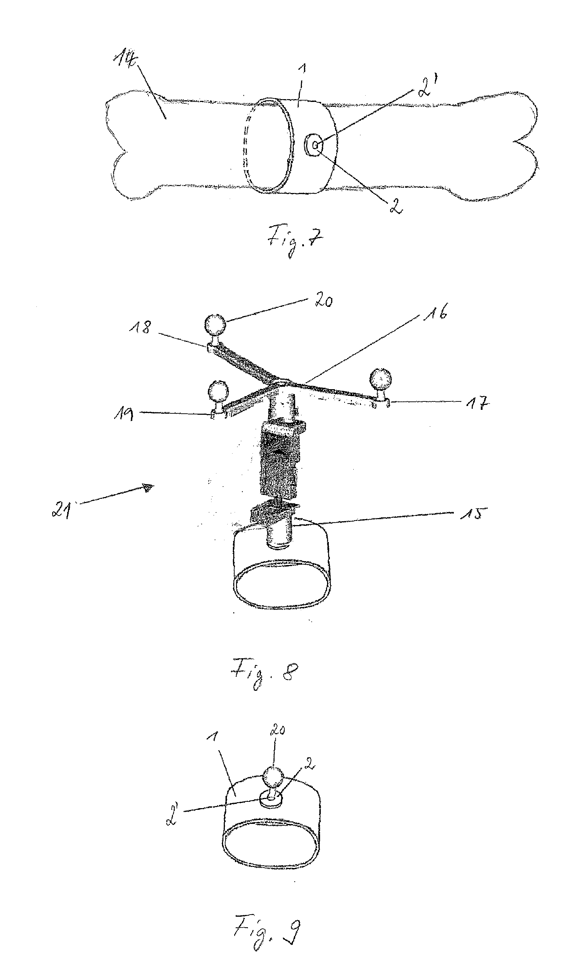

Wie aus Figur 1 erkennbar ist, erfolgt hier der Halt des Anbringteils 1 an dem Gegenstand 3 formschlüssig. Vorteilhaft kann die Marker-Anbringvorrichtung 6 wie in Figur 1 ein Anbringteil 1 eine Mantelfläche, z. B. in Form einer schlauchförmigen bzw. zylindrischen Hülse, oder eine offene Fläche wie weiter oben definiert besitzen. Ferner kann das Anbringteil 1 aber auch in Form eines Riemens oder Rings wie in Figur 2 ausgestaltet sein. Vorzugsweise ist das Anbringteil 1 wie in Figur 5 wenigstens teilweise netzförmig ausgebildet. Wie in Figuren 2 bis 4 angedeutet, kann das Anbringteil 1 auch eine Schließvorrichtung 7 (z.B. einen Einrastverschluss bzw. Klickverschluss, einen Klettverschluss, einen Druckknopfverschluss, ein einem Gurtschloss ähnliche oder gleiche Einrichtung, eine Gewindeverbindung, einen Nagels, eine Schraube, eine Schweißnaht und /oder eine Klebenaht) beinhalten, die den kraftschlüssigen und/oder reibschlüssigen und/oder formschlüssigen Sitz der Marker-Anbringvorrichtung 6 an dem Gegenstand 3 unterstützt und wenigstens zwei offene Enden des Anbringteils 1 miteinander (z.B. in einer Mantelfläche) verbindet. Hierdurch kann bei der Anpassung des Anbringteils 1 an die gegebene Oberfläche 5 eines Gegenstandes 3 eine zusätzliche mechanische Spannung an das Anbringteil 1 angelegt werden, die dem kraftschlüssigen bzw. reibschlüssigen Sitz der Marker-Anbringvorrichtung 6 an dem Gegenstand 3 dient. Ein rutschfester Sitz des Anbringteils 1 an dem Gegenstand 3 kann dadurch unterstützt werden, dass das Material des Anbringteils 1 auf der Oberfläche 4 des Anbringteils 1, die unmittelbar an der gegebenen Oberfläche 5 eines Gegenstandes 3 anliegt, mit einer rutschhemmenden Beschichtung 8 versehen ist. Diese rutschhemmende Beschichtung 8 kann insbesondere so ausgelegt sein, dass sie die Oberflächenbeschaffenheit des Gegenstands 3, an den die Marker-Anbringvorrichtung 6 angebracht werden soll, berücksichtigt. Die rutschhemmende Schicht 8 kann also an die Gleit- und Haftreibungskoeffizienten der gegebenen Oberfläche 5, deren chemische Beschaffenheit (z.B. zur Vermeidung unerwünschter Reaktion zwischen gegebener Oberfläche 5 und rutschhemmende Beschichtung 8), und/oder an die medizinischen, physiologischen und/oder hygienischen Anforderungen der gegebenen Oberfläche 5 in dem Fall, dass der Gegenstand 3 eine anatomische Körperstruktur 14 bzw. ein Implantat 14 (wie in Figur 7) darstellt, angepasst sein. Insbesondere erhöht die rutschhemmende Schicht 8 die Haftreibung zwischen der Oberfläche 5 und dem Anbringteil 1 gegenüber einer Ausführurtgsform, in der die rutschhemmende Schicht 8 nicht verwendet wird. Ein rutschfester Sitz des Anbringteils 1 kann auch dadurch unterstützt werden, dass die Marker-Anbringvorrichtung 6 ein Rutschverhinderungsmittel, z.B. eine aus einer der Oberflächen des Anbringteils 1 herausragende (etwa dorn- oder keilförmige und/oder abgerundete) Erhebung 9 (wie in Figuren 4, 6a und 6b dargestellt), einen Nagel oder eine Schraube 13, beinhaltet, das aus einer der Oberflächen 4, 24 des Anbringteils 1 herausragt und eine feste Verbindung zwischen der Marker-Anbringvorrichtung 6 und dem Gegenstand 3 durch Versenken bzw. Einschrauben des Nagels bzw. der Schraube 13 in den Gegenstand 3 bzw. die Oberfläche 5 des Gegenstandes gewährleistet. Vorzugsweise kann auch eine Vielzahl 10 von Erhebungen verwendet werden. Die aus einer der Oberflächen des Anbringteils 1 herausragende (etwa dorn- oder keilförmige und/oder abgerundete) Erhebung 9 kann dabei direkt (wie in Figur 6a) oder über ein Zwischenstück 11 (wie in Figur 6b) mit dem Anbringteil 1 verbunden sein. Vorteilhaft kann ein rutschfester Sitz des Anbringteils durch Verwendung einer aufgerauten Oberfläche 4 unterstützt werden.

In einer weiteren Ausführungsform der Erfindung kann wenigstens ein Bereich bzw. Randbereich 1' des Anbringteils 1 ein Material beinhalten, das anders ist als das Material, das wenigstens eine andere Region des Anbringteils 1 beinhaltet. Unter einer anderen Region wird hierbei insbesondere eine Region verstanden, die sich (beispielsweise bezüglich der Maße des Anbringteils 1) entfernt von dem Randbereich 1' befindet. Der Randbereich 1' kann auch den Bereich des Anbringteils 1 beinhalten, an dem sich die Schließvorrichtung 7 befindet bzw. an den die Schließvorrichtung 7 angefügt wird. Insbesondere kann das Material im Randbereich 1' des Anbringteils 1 bei gleichen äußeren Bedingungen weniger verformbar bzw. weniger leicht, d.h. schwerer (unter größerem Kraftaufwand) an wenigstens an eine gegebene Oberfläche 5 anpassbar sein als Material in wenigstens einer anderen Regionen des Anbringteils 1 Material im Randbereich 1' des Anbringteils 1 kann beispielsweise 5 %, 10 %, 100 % oder 200 % härter sein als Material in einer anderen Region des Anbringteils 1. Dies kann z.B. dazu dienen, dass ein in der Rohform und/oder im angeformten Zustand härteres Material im Randbereich 1' des Anbringteils 1 Beschädigungen am Anbringteil 1 und/oder Gegenstand 3 während des Anpassungsvorgangs oder während der laufenden Operation vorbeugt. Außerdem kann dadurch während des Anpassungsvorganges verhindert werden, dass eine für einen kraftschlüssigen und/oder reibschlüssigen und/oder formschlüssigen Sitz der Marker-Anbringvorrichtung 6 an dem Gegenstand 3 ungünstige Form des Anbringteils 1 plastisch modelliert wird. Das Material im Randbereich 1' kann die Herstellung bestimmter Oberflächenformen des Anbringteils 1 bei der Anpassung an den Gegenstand 3, 14 unterstützen, indem im plastisch verformbaren Zustand das Material des Anbringteils 1 wenigstens einen Randbereich 1' mit einer vorgegebenen, nicht modellierbaren Struktur und wenigstens einen anderen modellierbaren Bereich des Anbringteils 1 beinhaltet. Material im Randbereich 1, kann auch beispielsweise 10 %, 50 %, 100% dicker sein als als Material in wenigstens einer anderen Region des Anbringteils 1. Dadurch wird das Anbringteil 1 insgesamt steifer und stabiler. Wie in Figur 8 dargestellt, besteht eine Markervorrichtung, 21 die zur Verbindung mit der erfindungsgemäßen Marker-Anbringvorrichtung 6 vorgesehen ist, aus einem Anschlussstück 15 und einem mehrarmige Halter 16, an dem sich drei Adapter 17, 18, 19 für Markerelemente 20 befinden, die z.B. elektromagnetische Strahlung reflektieren können und dadurch die OP-Navigation im Rahmen der Image Guided Surgery (IGS) ermöglichen. Jeder Bestandteil einer solchen Markervorrichtung 21 kann dabei aus einem Metall und/oder einem Kunststoff hergestellt sein. Bei der Verbindung mit der erfindungsgemäßen Marker-Anbringvorrichtung 6 ist daher besonders zu beachten, dass die mechanischen Eigenschaften der Materialien von Markervorrichtung 21 und Marker-Anbringvorrichtung 6 dergestalt sind, dass sie unter mechanischer Belastung sich nicht gegenseitig beschädigen. Dazu werden vorzugsweise Materialien in der Markervorrichtung 21 und der Marker-Anbringvorrichtung 6 wenigstens an einer Berührungsstelle zwischen Markervorrichtung 21 und Marker-Anbringvorrichtung 6 verwendet, deren Härte gleich ist oder in einer gleichen Größenordnung liegt. Ferner ist zu beachten, dass die Materialien sich nicht gegenseitig chemisch beeinflussen (z.B. durch eine elektrochemische Reaktion, wenn beide Materialien aus unterschiedlichen Metallen hergestellt sind).

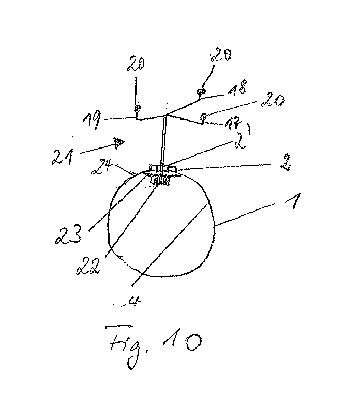

Figur 10 zeigt, wie der Sitz der Markervorrichtung an der Marker-Anbringvorrichtung stabilisiert werden kann: Indem man auf der Oberfläche 4 der Marker-Anbringvorrichtung, die der Oberfläche 24 der Marker-Anbringvorrichtung, auf der die Markervorrichtung 21 angebracht ist bzw. die auf die Markervorrichtung 21 zeigt bzw. weist, entgegengesetzt ist, eine Gegenhalterung (z.B. eine Schraubenmutter 21 mit Beilagscheibe 23) anbringt, kann z.B. ein Wackeln der Markervorrichtung 21 vermindert werden. Außerdem kann man dadurch einem Bewegen der Markervorrichtung 21 in einer Richtung senkrecht zu dem Flächenstück des Anbringteils 1, an dem die Markervorrichtung 21 angebracht ist, entgegenwirken. Die Gegenhalterung kann beispielsweise aus Metall oder einem Kunststoff ausgebildet sein und plattenförmig bzw. in Form in einer handelsüblichen Beilagscheibe bzw. scheibenförmig, sphärisch oder in Form eines Parallelepipeds oder eines Polyeders oder einer Mutter bzw. Schraubenmutter ausgestaltet sein und eine Materialdicke mit einer Untergrenze von 0,5 mm oder 1 mm oder 2 mm und/oder eine Obergrenze von 2 mm oder 5 mm oder 1 cm aufweisen, sollte jedoch (z. B. durch ein Schraubengewinde oder eine stoffschlüssige Verbindung, etwa eine Klebeverbindung) mit dem Anschlussstück 15 (z.B. ortsfest oder lösbar) verbunden sein. Diejenige Oberfläche der Gegenhalterung, die den festen Sitz der Markervorrichtung 21 an dem Anbringteil 1 unterstützt, kann beispielsweise einen Flächeninhalt mit einer Untergrenze von z.B. 4 mm² oder 10 mm² oder 1 cm² und/oder einer Obergrenze von z.B. 1 cm² oder 10 cm² oder 40 cm² besitzen. Ferner kann die Gegenhalterung aus einem flexiblen und/oder elastischen Material (etwa aus einem Gummi oder einem Kunststoff) und/oder einem mechanisch festen Material (z.B. einem Metall nach Art einer handelsüblichen Beilagscheibe) hergestellt sein.

Die feste Verbindung zwischen der Markervorrichtung 21 und Marker-Anbringvorrichtung 6 kann mittels einer in der Befestigungsmöglichkeit 2 beinhalteten mechanischen Halteverbindung 2' bzw. durch eine kraftschlüssige und/oder reibschlüssige und/oder formschlüssige und/oder stoffschlüssige Verbindung hergestellt werden. Eine solche mechanische Halteverbindung 2' bzw. eine solche kraftschlüssige und/oder reibschlüssige und/oder formschlüssige und/oder stoffschlüssige Verbindung kann eine Niete wie in einem handelsüblichen Druckknopfverschluss, einen Nagel, eine Schraube, eine Schweißnaht, eine Klebenaht, einen Klettverschluss und/oder einen Einrastmechanismus wie in den Figuren 2 bis 6b beinhalten. Vorzugsweise kann eine Kombination dieser mechanischen Halteverbindungen bzw. Verbindungsarten angewandt werden. Jedenfalls soll die mechanische Halteverbindung 2 formschlüssig und/oder kraftschlüssig und/oder reibschlüssig und/oder stoffschlüssig sowie insbesondere ortsfest wirken und insbesondere lösbar sein. Die Befestigungsmöglichkeit kann ferner beispielsweise den Rand eines Loches in einer Oberfläche des Anbringteils 1 beinhalten.

Figur 7 zeigt eine Marker-Anbringvorrichtung 6, die an einer anatomischen Körperstruktur 14 oder einem Implantat 14 anbringbar ist. In letzterem Fall ist darauf zu achten, dass sie leicht im Verformungszustand an die anatomische Körperstruktur 14 bzw. das Implantat 14 anformbar ist und der kraftschlüssige und/oder reibschlüssige und/oder formschlüssige Sitz der Marker-Anbringvorrichtung 6 so erzeugt wird, dass an der anatomischen Körperstruktur 14 keine vermeidbaren Schäden verursacht werden. Vorzugsweise wird hierzu anstelle der oben genannten herausragenden Erhebung 9, 10 ein rutschfester Sitz des Anbringteils 1 an der anatomischen Körperstruktur 14 bzw. dem Implantat 14 durch einen insbesondere physiologisch verträglichen Klebstoff (der z.B. Silikonpolymere beinhaltet) also stoffschlüssig_hergestellt, der zwischen der an der Körperstruktur 14 bzw. dem Implantat 14 zugewandten Oberfläche 4 des Anbringteils 1 und der Körperstruktur 14 bzw. dem Implantat 14 selbst eingebracht wird. Im Interesse einer Rationalisierung des Operationsverfahrens und der Vermeidung zusätzlicher Eingriffe kann die Marker-Anbringvorrichtung 6 auch so gestaltet sein, dass sie aus einem vom menschlichen und/oder tierischen Körper resorbierbaren Material (z.B. Polylaktide und/oder Polyglykolid) besteht. Dann kann nach dem Ende der Operation die Marker-Anbringvorrichtung 6 an einer anatomischen Körperstruktur 14 bzw. einem Implantat verbleiben, ohne entfernt werden zu müssen.

Beispielsweise kann an der Marker-Anbringvorrichtung 6 nicht nur eine Markervorrichtung 21 angebracht werden, die eine Halterung für eine Vielzahl von Markern 20 darstellt. Vielmehr kann wie in Figur 9 auch die Markervorrichtung in Form eines einzelnen Markers 20 der Marker-Anbringvorrichtung 6 befestigt werden. Für die dann herzustellende Verbindung zwischen Marker 20 und Marker-Anbringvorrichtung 6 kann ein mechanische Halteverbindung 2' an der Befestigungsmöglichkeit 2 verwendet werden, wie sie weiter oben vorgeschlagen wurde.

## Patentansprüche

1. Marker-Anbringvorrichtung (6) zum Anbringen einer Markervorrichtung (21) an einem Gegenstand, insbesondere an einem medizinischen Instrument (3) und/oder einer anatomischen Körperstruktur (14) und/oder einem Implantat (14), mit einer Befestigungsmöglichkeit (2) für eine Markervorrichtung (21) und einem Anbringteil (1) zum Anbringen der Marker-Anbringvorrichtung (6),
**dadurch gekennzeichnet, dass**
das Anbringteil (1) verformbar ist, um mit dem Gegenstand eine mechanische Verbindung einzugehen.

2. Marker-Anbringvorrichtung (6) nach Anspruch 1, **dadurch gekennzeichnet, dass** das Anbringteil (1) aus Material hergestellt ist, das mittels Energiezufuhr plastisch verformbarer wird.

3. Marker-Anbringvorrichtung (6) nach einem der vorhergehenden Ansprüche, **dadurch gekennzeichnet, dass** das Anbringteil (1) eine Schließvorrichtung (7) zur Unterstützung des Sitzes der Marker-Anbringvorrichtung (6) an dem Gegenstand (3, 14) und/oder wenigstens ein rutschhemmendes Mittel (9) zur Unterstützung des Sitzes des Anbringteils (1) an dem Gegenstand (3, 14) beinhaltet.

4. Marker-Anbringvorrichtung (6) nach einem der vorhergehenden Ansprüche, **dadurch gekennzeichnet, dass** ein Randbereich (1') des Anbringteils (1) aus einem anderen, insbesondere härteren, Material und/oder dicker als wenigstens eine andere Region des Anbringteils (1) ausgebildet ist, wobei das Material im Randbereich (1') des Anbringteils (1) schwerer, d.h. unter größerem Kraftaufwand, plastisch verformbar ist als das Material in wenigstens einer anderen Regionen des Anbringteils (1).

5. Marker-Anbringvorrichtung (6) nach einem der vorhergehenden Ansprüche, **dadurch gekennzeichnet, dass** auf der Oberfläche (4) des Anbringteils (1), die der Oberfläche (24) des Anbringteils (1), die auf die Markervorrichtung (21) zeigt, entgegengesetzt ist, eine Gegenhalterung (22, 23) vorgesehen ist.

6. Marker-Anbringvorrichtung (6) nach einem der vorhergehenden Ansprüche, **dadurch gekennzeichnet, dass** die Befestigungsmöglichkeit (2) für die Markervorrichtung (21) an der Marker-Anbringvorrichtung (6) einer mechanischen Halteverbindung(2') zur mechanischen Verbindung der Marker-Anbringvorrichtung (6) mit der Markervorrichtung (21) beinhaltet.

7. System aus einer Marker-Anbringvorrichtung (6) nach einem der Ansprüche 1 bis 8 und einem medizinischen Gerät (3) und/oder einem Implantat (14), das mit der Markeranbringvorrichtung (6) verbunden ist..

8. System aus einer Marker-Anbringvorrichtung (6) nach einem der Ansprüche 1 bis 6 oder dem System nach Anspruch 9 und einer Markervorrichtung (21).

9. Verfahren zum Befestigen einer Marker-Anbringvorrichtung (6) nach einem der Ansprüche 1 - 6 an einem Gegenstand, insbesondere an einem medizinischen Instrument (3) und/oder einer anatomischen Körperstruktur (14) und/oder einem Implantat (14), mit zumindest einem der folgenden Schritte:
- Einwirkung (S2, S101, S203, S304) von Energie auf das Material des Anbringteils 1, um das Material des Anbringteils (1) plastisch verformbarer (S3, S102) zu machen;
- Anformen (S4, S104, S302, S202) des Anbringteils (1) an wenigstens eine gegebene Oberfläche (5) des Gegenstandes (3, 14).

10. Verfahren nach Anspruch 10, **dadurch gekennzeichnet, dass** es ferner folgenden Schritt beinhaltet:
- fester machen (S5, S105, S306, S205) des Anbringteils 1.

11. Verfahren zum Befestigen einer Marker-Anbringvorrichtung (6) nach einem der Ansprüche 1 - 6 an einem Gegenstand, insbesondere an einem medizinischen Instrument (3) und/oder einer anatomischen Körperstruktur (14) und/oder einem Implantat (14), mit zumindest einem der folgenden Schritte:
- Einwirkung (S2, S101, S203, S304) von Energie auf das Material des Anbringteils 1, um das Material des Anbringteils (1) mechanisch fester (S204, S305) zu machen;
- Anformen (S4, S104, S302, S202)des Anbringteils (1) an wenigstens eine gegebene Oberfläche (5) des Gegenstandes (3, 14).

12. Verfahren zum Entfernen einer Marker-Anbringvorrichtung (6) nach einem der Ansprüche 1 - 6 von einem Gegenstand, insbesondere von einem medizinischen Instrument (3) und/oder einer anatomischen Körperstruktur (14) und/oder einem Implantat (14), mit zumindest einem der folgenden Schritte:
- Einwirkung (S401) von Energie auf das Material des Anbringteils (1), um das Anbringteil (1) plastisch verformbarer zu machen;
- Räumliches Entfernen (S404) des Anbringteils (1) von dem Gegenstand (3, 14).

13. Verfahren nach einem der Ansprüche 12 bis 13, **dadurch gekennzeichnet, dass** die Einwirkung von Energie (S2, S101, S203, S304, S401) die Einwirkung mechanischer Schwingungen bzw. Wellen und/oder thermischer Energie und/oder elektromagnetischer Strahlung und/oder einer chemischen Reaktion und/oder elektrischen Stroms beinhaltet.

14. Verwendung einer Marker-Anbringvorrichtung (6) nach einem der Ansprüche 1 bis 6, wobei die Anbringvorrichtung (6) an einem Gegenstand, insbesondere an einem medizinischen Instrument (3) und/oder einer anatomischen Körperstruktur (14) und/oder einem Implantat (14), insbesondere gemäß einem Verfahren nach einem der Ansprüche 9 bis 11 oder 13 befestigt und/oder insbesondere gemäß dem Verfahren nach einem der Ansprüche 12 bis 13 entfernt wird.
